# EUROPÄISCHE PATENTSCHRIFT

(11) **EP 2 524 665 B1**
(45) Veröffentlichungstag und Bekanntmachung des Hinweises auf die Patenterteilung: **02.04.2014**
(21) Anmeldenummer: 12002291.8
(22) Anmeldetag: 29.03.2012
(51) Int. Cl.: A61B 19/08, A61F 7/00

(54) **Kunststoffauskleidung für temperierbare Schalen**
Plastic cladding for temperable shells
Habillage en plastique pour coques pouvant être tempérées

(30) Priorität: 20.05.2011 DE 202011101217 U
(43) Veröffentlichungstag der Anmeldung: 21.11.2012
(73) Patentinhaber: PEMAX Kunststoff GmbH, 70563 Stuttgart (DE)
(72) Erfinder: Bahr, Ulrich, 72290 Lossburg (DE)
(74) Vertreter: Reinhardt, Harry

(56) Entgegenhaltungen:
- WO-A1-98/58594
- US-A- 4 889 135
- US-A- 5 435 322
- US-A1- 2004 045 954
- US-A1- 2004 194 673

## Beschreibung

### Gebiet der Erfindung

Die Erfindung betrifft eine Kunststoffauskleidung für temperierbare Schalen, insbesondere für Operationsschüsseln, nach dem Oberbegriff des Anspruches 1.

### Stand der Technik

Bei einer Vielzahl von chirurgischen Operationen und Eingriffen in Krankenhäusern und Kliniken wird am Operationstisch eine Stahlschüssel mit Kochsalz- oder Medikamentenlösungen bereitgestellt. Diese Kochsalzlösung dient zum Auswaschen von Wunden und zu sonstigen Reinigungszwecken bzw. zum Befeuchten von Reinigungstüchern. Die Kochsalzlösung wird in einer sterilen Metallschüssel bereitgestellt, die auf einer Wärmeplatte mit einem fahrbaren Unterbau bereitgehalten und auf Körpertemperatur temperiert ist, damit die Salzlösung ebenfalls auf Körpertemperatur angewärmt ist. Nach jeder Operation muss die Stahlschüssel gereinigt, desinfiziert, verpackt und sterilisiert werden, was einen erheblichen Material- und Kostenaufwand erfordert. Der Unterbau ist ebenfalls nicht steril und kann eine Infektionsquelle darstellen, so dass er häufig mit zweckentfremdeten OP-Bezügen abgedeckt wird, was allerdings zu einer Wärmeisolation zwischen Stahlschüssel und Wärmeplatte führen kann.

Aus der dem Oberbegriff des Anspruches 1 zugrunde liegenden WO 98/58594 A1 ist eine Kunststoffauskleidung für temperierbare Schalen wie insbesondere Operationsschalen bekannt, die auf einem Unterbau gelagert wird. Die lösbar an der Schale aufgenommene Kunststoffauskleidung ist an die Form der Schale angepasst und kleidet diese damit aus. Außerhalb eines in der Schale befindlichen Schalenbereichs ist die Auskleidung am Unterbau der Schale festlegbar und weist einen Schürzenteil zur Abdeckung des Unterbaus auf. Der Schalenbereich ist dabei durch wenigstens ein der Form der Schale angepasstes Formteil gebildet, das am Schürzenteil angeformt ist. Im Schalenbereich selbst ist die Kunststoffauskleidung mehrlagig und weist eine Wärmeschutzschicht auf.

In der US 5,435,322 A wurde vorgeschlagen, für Operationsschüsseln Einsatzbeutel aus Kunststoff zu verwenden, an denen ein Formteil zum Einsatz in die Schüssel angeformt ist (siehe auch US 5,457,962 A). Die Stahlschüssel wird also mit Kunststofffolien oder -beuteln ausgekleidet, die nach jeder Operation entsorgt werden können. Die eingangs genannten Probleme konnten damit zwar behoben werden, jedoch leiden derartige Auskleidungen an zu geringer Temperaturbeständigkeit, gelegentlicher Undichtigkeit, ungenauer Passform, schlechter Temperaturleitfähigkeit, der Möglichkeit eines Verklebens des Kunststoffs mit der Schale durch Erhitzung und mechanischer Beschädigung der schwachen Folie.

Dies gilt auch für die aus der DE 20 2006 007 675 U1 bekannte Auskleidung, die nur aus einer Folie ohne Formteil besteht.

Mitunter neigen die Verwender derartiger Auskleidungen auch dazu, die Auskleidung längere Zeit ohne Flüssigkeit in den aufgeheizten Schalen stehen lassen. Da in der Aufheizphase am Boden der Schüssel jedoch Temperaturen entstehen, die wesentlich höher als die vorgesehenen 36-40°C sind, kann es je nach Kunststoff zu einem Erweichen kommen und Undichtigkeiten können auftreten, was zu einer Unsterilität führen kann.

### Zusammenfassung der Erfindung

Ausgehend von diesem Stand der Technik liegt der vorliegenden Erfindung die Aufgabe zugrunde, eine bessere Wärmeübertragung von der Schale auf die Flüssigkeit unter Verwendung einer Kunststoffauskleidung zu erreichen.

Diese Aufgabe wird durch eine Kunststoffauskleidung mit den Merkmalen des Anspruches 1 gelöst.

Die Kunststoffauskleidung umfasst wenigstens ein der Form der Schale angepasstes Formteil, an das ein den Unterbau bedarfsweise zumindest teilweise abdeckendes Schürzenteil angeformt ist. Durch die damit erzielte Passgenauigkeit, kann einerseits nahezu ein die Wärmeübertragung steigernder unmittelbarer Formschluss zwischen Schale und Kunststoffauskleidung erreicht werden, ohne die einfache Bedienbarkeit durch Entnahme der Kunststoffauskleidung nach Gebrauch zu beeinträchtigen. Damit wird eine Bildung von Falten im Schalenbereich vermieden, was auch dazu beiträgt, dass eine Verletzung der Kunststoffauskleidung in diesem Bereich zumindest aufgrund von Falten nicht möglich ist. Der an den Rand des Schalenbereichs angeschweißte Schürzenteil kann nach Einlegen der Auskleidung in die Schale seitlich nach unten gezogen werden, um den Unterbau steril abzudecken, sofern dies erforderlich ist. Gleichzeitig ergibt sich ein ansprechendes Äußeres, da die Formteile optisch ansehnlich sind und insgesamt durch die optimale Passform die qualitativ hochwertige Ausführung unterstrichen wird. Im Schalenbereich sind vorzugsweise oberhalb einer Wärmeschutzschicht, die als Aluminium-Schicht ausgebildet ist, mehrere Schichten von Kunststoff-Folien vorgesehen, die ineinander liegen und damit eine Mehrlagigkeit bilden. Durch diese Mehrlagigkeit ist selbst bei einer mechanischen Beschädigung an der Innenschicht immer noch eine Dichtigkeit des Produkts gewährleistet. Bedenkt man, dass gerade im OP-Bereich regelmäßig auch Skalpells oder andere spitze Gegenstände verwendet werden, die auch unter Umständen in die Flüssigkeit der Schale eingetaucht werden, ist dies ein nicht zu verachtender Vorteil.

Die Kunststoffauskleidung ist vorzugsweise mit einem eingeschweißten Flachboden aus einem Laminat Aluminium/Polyethylen versehen, wobei die Kontaktseite zur Schale hin aus Aluminium besteht. Das steife Aluminium-Laminat passt sich beim Befüllen der Schalen-Auskleidung gut der Schalenform an und dadurch wird eine optimale Wärmeleitfähigkeit hergestellt. Das Aluminium-Laminat ist gegen die gerätebedingten hohen Temperaturschwankungen völlig unempfindlich

Der Aluminium-Rundzuschnitt an der Bodenaußenseite der Auskleidung weist in Richtung des Schaleninnenbodens keinerlei Schweißnähte auf und ist plan, so daß hier auch keine Undichtigkeit durch eine hitzebedingte Beschädigung von Schweißnähten auftreten kann. Gleichsam erleichtert diese Anordnung eine bessere Positionierung der Formteile im Schalenbereich.

Wird die Kunststoffauskleidung im Schürzenteil transparent, also z.B. aus transparenter Kunststofffolie ausgebildet, ist die Sicht auf den Ständer und damit auch auf die üblicherweise dort angebrachte Temperaturregelung frei.

Weitere Vorteile ergeben sich aus den Unteransprüchen und der nachfolgenden Beschreibung eines Ausführungsbeispiels.

### Kurzbeschreibung der Figuren

Im Folgenden wird die Erfindung anhand von in den beigefügten Figuren dargestellten Ausführungsbeispielen näher erläutert. Es zeigen:
- Fig. 1: eine dreidimensionale Ansicht der Kunststoffauskleidung,
- Fig. 2: die an der Schale und dem Unterbau angebrachte Kunststoffauskleidung,
- Fig. 3: eine Ansicht gemäß Fig. 1 in einem weiteren Ausführungsbeispiel.

### Beschreibung bevorzugter Ausführungsbeispiele

Bevor die Erfindung im Detail beschrieben wird, ist darauf hinzuweisen, dass sie nicht auf die jeweiligen Bauteile der Vorrichtung sowie die jeweiligen Verfahrensschritte beschränkt ist, da diese Bauteile und Verfahren variieren können. Die hier verwendeten Begriffe sind lediglich dafür bestimmt, besondere Ausführungsformen zu beschreiben und werden nicht einschränkend verwendet. Wenn zudem in der Beschreibung oder in den Ansprüchen die Einzahl oder unbestimmte Artikel verwendet werden, bezieht sich dies auch auf die Mehrzahl dieser Elemente, solange nicht der Gesamtzusammenhang eindeutig etwas Anderes deutlich macht.

Die Figuren zeigen Kunststoffauskleidungen für temperierbare Schalen, wie sie nicht nur, aber insbesondere in Krankenhäusern und Kliniken verwendet wird. Der Einsatzzweck der Kunststoffauskleidung ist jedoch nicht auf derartige Schalen beschränkt, sondern grundsätzlich für sämtliche Aufnahmegefäße geeignet, in denen Stoffe und Flüssigkeiten temperiert werden können.

Die gezeigten Ausführungsbeispiele einer Kunststoffauskleidung für eine temperierbare Operationsschüssel zeigt einen Verwendungszweck, wie er in Krankenhäusern Anwendung findet, um z.B. Kochsalzlösungen während chirurgischer Operationen vorzuhalten. Durch den Unterbau 5 wird die Schale 4 temperiert, in der eine zeichnerisch nicht dargestellte Flüssigkeit aufgenommen ist. Um die Reinigung, Desinfektion und Sterilisierung zu vereinfachen, bzw. die Kosten hierfür zu reduzieren, wird eine herausnehmbare Kunststoffauskleidung verwendet, die im Folgenden näher erläutert wird. Ziel ist damit, einerseits den Sterilisationsaufwand zu verringern und andererseits auf günstige Weise eine effektive und schnelle Entfernung des Inhalts der Schale zu ermöglichen. Zu diesem Zweck ist die Kunststoffauskleidung lösbar in der Schale 4 aufgenommen und an die Form der Schale 4 angepasst. Die Befestigung der Kunststoffauskleidung erfolgt außerhalb eines in der Schale befindlichen Schalenbereichs 4a und damit an der Schale selbst, d.h. an ihrer Unterseite oder an ihrem Unterbau 5. Die Kunststoffauskleidung besitzt dazu ferner ein diesen Bereich außerhalb des Schalenbereichs 4a zumindest teilweise schürzenden Schürzenteil 2 zur zumindest teilweisen Abdeckung von Schale 4 und/oder Unterbau 5. Eine derartige teilweise Abdeckung liegt bereits vor, wenn der Rand der Kunststoffauskleidung schürzenartig über die Außenseite der Schale geführt ist. Vorzugsweise wird jedoch ein Schürzenteil vorhanden sein, das zur sterilen Abdeckung des Unterbaus 5 dient.

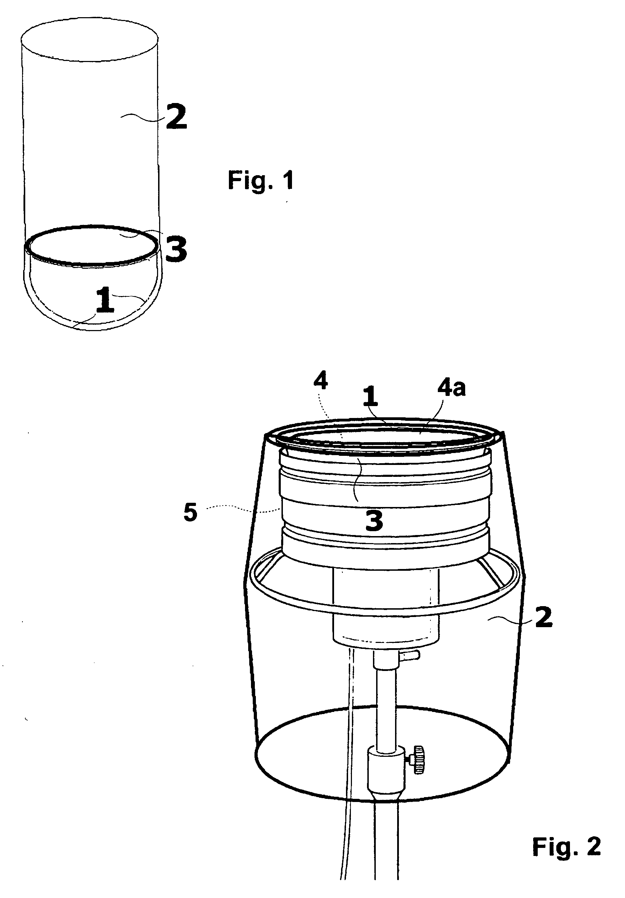

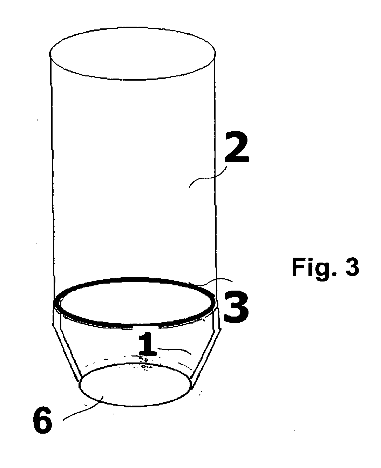

Gemäß Fig. 1 wird der Schalenbereich durch wenigstens ein der Form der Schale 4 angepasstes Formteil 1 gebildet, an das der Schürzenteil 2 angeformt ist. Vorzugsweise ist die Kunststoffauskleidung zumindest im Schalenbereich 4a mehrlagig, im ersten nicht erfindungsgemäßen Ausführungsbeispiel der Fig. 1 und 2 zweilagig, wobei als Kunststoff ein wärmeleitfähiger Kunststoff gewählt wird, der vorzugsweise hochreißfest und hochtemperaturbeständig ist. Ergänzend zu dem wenigstens einlagigen, vorzugsweise mehrlagigen Formteil 1 kann im zweiten Ausführungsbeispiel der Fig. 3 erfindungsgemäß eine Wärmeschutzschicht 6 vorgesehen werden. Durch die Verwendung von Formteilen 1 ergibt sich ein ausgesteifter Bodenbereich im Schalenbereich 4a, wobei die Kunststoffauskleidung durch ein z.B. im Thermoforming-Verfahren hergestelltes Tiefziehteil gebildet werden kann, das genau der Form der Schale 4 entspricht. Durch die genau der Schale 4 angepasste Form wird eine bessere Wärmeübertragung von der Schale zur Flüssigkeit erreicht. Ebenso gibt es damit keine Bildung von Falten im Schalenbereich 4a mehr, so dass der Einsatz sauber an der Schaleninnenseite anliegt. Damit kann in diesem Bereich auch eine Verletzung der Kunststoffauskleidung zuverlässig unterbunden werden.

Das Formteil 1 ist im Schalenbereich 4a mehrlagig ausgebildet, so dass in diesem Bereich selbst bei einer mechanischen Beschädigung der Innenschicht immer noch eine Dichtigkeit des Produkts gewährleistet ist. Da vorzugsweise der Kunststoff so gewählt ist, dass eine hohe Durchstoß- und Reißfestigkeit erreicht wird, lassen sich mögliche Verletzungen der Kunststoffauskleidungen auch durch die Materialwahl zuverlässig vermeiden. Der Kunststoff ist ferner temperaturstabilisiert, so dass sein Wärmeverhalten als auch seine Wärmeleitfähigkeit für den bevorzugten Einsatzzweck in Verbindung mit temperierbaren Schalen optimiert ist.

Gemäß Fig. 2 besitzt die Kunststoffauskleidung vorzugsweise drei Teile, d.h. wenigstens zwei ineinanderliegende, den Schalenbereich 4a umfassende Formteile 1 sowie den vorzugsweise aus einer Kunststofffolie gebildeten Schürzenteil 2. Dieser Schürzenteil ist mit den Formteilen 1 z.B. durch eine Verschweißung 3 verbunden, wobei die Verschweißung 3 außerhalb des Schalenbereichs 4a zu liegen kommt. Damit sind einerseits im Schalenbereich 4a keinerlei Schweißnähte vorgesehen, an denen eine Undichtigkeit auftreten könnte. Andererseits kann die Verschweißung, die die Formteile 1 und den Schürzenteil 2 miteinander verbindet, leicht zur Orientierung bei der Positionierung der Kunststoffauskleidung in der Schale 4 genutzt werden. Sobald nämlich die Verschweißung 3 ringsum außerhalb der Schale 4 liegt, ist eine zuverlässige und einfache Positionierung sichergestellt.

Für den bevorzugten Einsatzzweck wird die Kunststoffauskleidung in einen vorgefertigten Beutel verbracht, so dass sie keimfrei sterilisiert in den Verkehr und damit in den OP gelangen kann. Dort werden die Formteile 1 in der Schale 4 positioniert, so dass das Formteil an der Schale passgenau anliegt, um eine möglichst gleichmäßige Übertragung der Temperatur der Schale auf die in der Kunststoffauskleidung befindliche Flüssigkeit sicherzustellen. Sobald die Formteile 1 in der Schale 4 positioniert sind, kann der Schürzenteil nach unten gezogen werden, um den Außenbereich und Unterbau 5 abzudecken.

Im Ausführungsbeispiel der Fig. 3 wir zusätzlich zu dem wenigstens einlagigen Formteil 1 eine Wärmeschutzschicht 6 wenigstens im Schalenbereich 4a und vorzugsweise wenigstens im Bereich des Innenbodens des Schalenbereichs 4a vorgesehen. Es hat sich nämlich herausgestellt, dass Anwender mitunter die Auskleidung längere Zeit ohne Flüssigkeit in den aufgeheizten Schalen stehen lassen. In der Aufheizphase entstehen am Schalenboden Temperaturen von bis zu 80° C, was je nach Kunststoff zu einem starken Erweichen und ggf. zu Undichtigkeiten führen kann. Um dagegen gewappnet zu sein, wird die Wärmeschutzschicht 6 z.B. auf die äußere Folienschicht bzw. auch das äußere Formteil 1 z.B. als Aluminium-Verbundfolie aufgesiegelt. Vorzugsweise sind damit im Schalenbereich 4a oberhalb der Aluminium-Schicht mehrere Schichten von Kunststoff-Folien vorgesehen, die ineinander liegen und damit eine Mehrlagigkeit bilden. Dadurch kann z.B. eine Temperaturbeständigkeit erreicht werden, so dass die verwendeten Kunststoffe erst bei dauerhaften Temperaturen von 120 °C am Schalenboden und nachdem das Wasser bereits 20 Minuten kochte, in einen riskanten Bereich vorstoßen. Somit ist das Produkt auf günstige Weise zuverlässig auch gegen maximal erreichbare Temperaturen resistent.

Der Aluminium-Rundzuschnitt an der Bodenaußenseite der Auskleidung weist in Richtung des Schaleninnenbodens keinerlei Schweißnähte auf und ist plan, so dass hier auch keine Undichtigkeit durch eine hitzebedingte Beschädigung von Schweißnähten auftreten kann. Die Kunststoffauskleidung ist vorzugsweise mit einem eingeschweißten Flachboden aus einem Laminat Aluminium/Polyethylen versehen, wobei die Kontaktseite zur Schale hin aus Aluminium besteht. Das steife Aluminium-Laminat passt sich beim Befüllen der Schalen-Auskleidung gut der Schalenform an und dadurch wird eine optimale Wärmeleitfähigkeit hergestellt. Das Aluminium-Laminat ist gegen die gerätebedingten hohen Temperaturschwankungen unempfindlich.

Die Kunststoffauskleidung ist im Schürzenteil 2 vorzugsweise transparent, also z.B. aus transparenter Kunststofffolie ausgebildet. Damit ist die Sicht auf den Unterbau und damit auch auf die üblicherweise dort angebrachte Temperaturregelung frei, die bedarfsweise auch durch die Folie hindurch bedient werden kann, so dass damit die Hygiene bis in diesen Bereich ohne weiteres gewährleistet ist.

## Patentansprüche

1. Kunststoffauskleidung für temperierbare Schalen (4), insbesondere für Operationsschalen, die auf einem Unterbau (5) gelagert sind, wobei die lösbar in der Schale (4) aufgenommene Kunststoffauskleidung an die Form der Schale (4) angepasst diese auskleidet und außerhalb eines in der Schale befindlichen Schalenbereichs (4a) an der Schale (4) oder deren Unterbau (5) festlegbar ist und ferner einen Schürzenteil (2) zur zumindest teilweisen Abdeckung des Unterbaus (5) aufweist, wobei der Schalenbereich durch wenigstens ein der Form der Schale (4) angepasstes Formteil (1) gebildet ist, an den der Schürzenteil (2) angeformt ist, wobei zumindest im Schalenbereich die Kunststoffauskleidung (4a) mehrlagig ist,
**dadurch gekennzeichnet, dass** die Kunststoffauskleidung (4a) im Bereich des Formteils (1) einen Aluminium-Zuschnitt als Wärmeschutzschicht (6) aufweist.

2. Kunststoffauskleidung nach Anspruch 1, **dadurch gekennzeichnet, dass** sie aus einem wärmeleitfähigen Kunststoff besteht.

3. Kunststoffauskleidung nach einem der Ansprüche 1 oder 2, **dadurch gekennzeichnet, dass** als Wärmeschutzschicht (6) eine Aluminium-Verbundfolie Sandwichboden-artig auf die Auskleidung aufgesiegelt ist.

4. Kunststoffauskleidung nach einem der vorhergehenden Ansprüche, **dadurch gekennzeichnet, dass** die Wärmeschutzschicht (6) ein in die Kunststoffauskleidung eingeschweißter Flachboden aus einem Aluminium/Polyethylen-Laminat ist, wobei das Aluminium als Kontaktseite zur Schale (4) hin zeigt.

5. Kunststoffauskleidung nach einem der vorhergehenden Ansprüche, **dadurch gekennzeichnet, dass** die Kunststoffauskleidung wenigstens zwei ineinanderliegende, den Schalenbereich (4a) umfassende Formteile (1) sowie den vorzugsweise aus Kunststofffolie gebildeten Schürzenteil (2) aufweist.

6. Kunststoffauskleidung nach einem der Ansprüche 3 bis 5, **dadurch gekennzeichnet, dass** die Verbindung, vorzugsweise die Verschweißung (3), der Wärmeschutzschicht (6) mit dem Schürzenteil (2) außerhalb des Innenbodens des Schalenbereichs (4a) erfolgt.

7. Kunststoffauskleidung nach einem der vorhergehenden Ansprüche, **dadurch gekennzeichnet, dass** sie zumindest im Schürzenteil (2) wenigstens teilweise transparent ist.

## Claims

1. Plastics covering for temperature-controllable dishes (4), in particular for surgical dishes which are mounted on a substructure (5), wherein the plastics covering which is releasably received in the dish (4) is conformed to the shape of the dish (4), covers said dish and can be fastened outside a dish region (4a) located in the dish, to the dish (4) or the substructure (5) thereof and also has a skirt portion (2) for at least partially covering the substructure (5), wherein the dish region is formed by at least one moulded part (1) conforming to the shape of the dish (4), and onto which the skirt portion (2) is formed, wherein at least in the dish region, the plastics covering (4a) is multiple-layered,
**characterized in that** the plastics covering (4a) has an aluminium blank in the region of the moulded part (1) as a thermal protection layer (6).

2. Plastics covering according to claim 1, **characterised in that** it consists of a thermally conductive plastics material.

3. Plastics covering according to one of the claims 1 or 2, **characterised in that** an aluminium composite film is sealed in the manner of a sandwich base onto the covering as a thermal protection layer (6).

4. Plastics covering according to one of the preceding claims, **characterised in that** the thermal protection layer (6) is a flat base made from an aluminium/polyethylene laminate welded into the plastics covering, wherein the aluminium is directed toward the dish (4) as a contact side.

5. Plastics covering according to one of the preceding claims, **characterised in that** the plastics covering has at least two moulded parts (1), lying within one another and encompassing the dish region (4a), as well as the skirt portion (2) which is preferably made from a plastics film.

6. Plastics covering according to one of the claims 3 to 5, **characterised in that** the connection, preferably the welding (3) of the thermal protection layer (6) to the skirt portion (2) is carried out outside the internal base of the dish region (4a).

7. Plastics covering according to one of the preceding claims, **characterized in that** it is at least partially transparent at least in the skirt portion (2).

## Revendications

1. Habillage en plastique pour des coques pouvant être tempérées (4), en particulier pour des coques pour intervention chirurgicale, qui sont stockées sur un bâti (5), dans lequel l'habillage en matière plastique reçu de manière amovible dans la coque (4) habille cette dernière d'une manière adaptée à la forme de la coque (4) et peut être fixé à la coque (4) ou à son bâti (5) à l'extérieur d'une zone de coque (4a) se trouvant dans la coque et comporte en outre une partie formant jupe (2) destinée à revêtir au moins partiellement le bâti (5), la zone de coque étant formée par au moins un élément moulé (1) adapté à la forme de la coque (4) et auquel se rattache la partie formant jupe (2), l'habillage en plastique (4a) étant au moins multicouche dans la zone de coque, **caractérisé en ce que** l'habillage en plastique (4a) comporte dans la zone de l'élément moulé (1) une section en aluminium en tant que couche de protection thermique (6).

2. Habillage en plastique selon la revendication 1, **caractérisé en ce qu'**elle est constituée d'une matière plastique conduisant la chaleur.

3. Habillage en matière plastique selon la revendication 1 ou 2, **caractérisé en ce qu'**en tant que couche de protection thermique (6), un film composite d'aluminium est scellé en sandwich au fond de l'habillage.

4. Habillage en plastique selon l'une des revendications précédentes, **caractérisé en ce que** la couche de protection thermique (6) est un fond plat en stratifié d'aluminium/polyéthylène soudé dans l'habillage en plastique, l'aluminium étant la face de contact tournée vers la coque (4).

5. Habillage en plastique selon l'une des revendications précédentes, **caractérisé en ce que** l'habillage en plastique comporte au moins deux éléments moulés (1) superposés entourant la zone de coque (4a) ainsi que la partie formant jupe (2) formée de préférence d'une feuille de matière plastique.

6. Habillage en plastique selon l'une des revendications 3 à 5, **caractérisé en ce que** la liaison, de préférence la soudure (3), de la couche de protection thermique (6) avec la partie formant jupe (2) s'effectue à l'extérieur du fond intérieur de la zone de coque (4a).

7. Habillage en plastique selon l'une des revendications précédentes, **caractérisé en ce qu'**elle est au moins partiellement transparente au moins dans la partie formant jupe (2).
